# EUROPEAN PATENT SPECIFICATION

(11) **EP 3 210 628 B1**
(45) Date of publication and mention of the grant of the patent: **05.06.2024**
(21) Application number: 17152093.5
(22) Date of filing: 21.07.2015
(51) Int. Cl.: A61K 47/02, A61K 47/26, A61K 9/08, C07H 23/00, A61K 31/185, A61K 31/7024, A61K 33/06

(54) **METHODS FOR FRUCTOBORATE COMPLEXES**
VERFAHREN FÜR FRUCTOBORATKOMPLEXE
PROCÉDÉS POUR FRUCTOBORATE COMPLEXES

(30) Priority: 29.08.2014 US 201414473870; 18.09.2014 LU 92551
(43) Date of publication of application: 30.08.2017
(62) Divisional of application: 15177703.4
(73) Proprietor: VDF Futureceuticals, Inc., Momence, Illinois 60954 (US)
(72) Inventor: HUNTER, John M., Momence, IL Illinois 60954 (US)
(74) Representative: Petty, Catrin Helen

(56) References cited:
- WO-A1-98/43652
- US-B2- 6 924 269
- M. MAKKEE ET AL: "Studies on borate esters III. Borate esters of D-mannitol, D-glucitol, D-fructose and D-glucose in water", RECUEIL DES TRAVAUX CHIMIQUES DES PAYS-BAS, vol. 104, no. 9, 2 September 1985 (1985-09-02), pages 230-235, XP055177536, ISSN: 0165-0513, DOI: 10.1002/recl.19851040905

## Description

This application claims the benefit of US patent application with the serial number 14/473870, filed 29 August, 2014, and of Luxembourg patent application with the serial number 92551, filed 18 September, 2014.

### Field of the Invention

The field of the invention is fructoborate complexes, and especially methods for production of such complexes with improved parameters. The specific example of a carbohydrate is the fructose and the specific example of a borocarbohydrate is the fructoborate in the present invention.

### Background of the Invention

The background description includes information that may be useful in understanding the present invention. It is not an admission that any of the information provided herein is prior art or relevant to the presently claimed invention, or that any publication specifically or implicitly referenced is prior art.

For many years, calcium fructoborate (CF) has been a nutritional supplement of interest with many potential medicinal and therapeutic applications. For example, CF has been shown to be an effective antioxidant (Scorei et. al., Biological Trace Element Research 107, no. 2 (2005): 127-34), to be effective against cancer (Scorei and Popa, 2010, Anti-Cancer Agents in Medicinal Chemistry 10, no. 4 (May 1, 2010): 346-51), and to be a relatively effective modality for reducing inflammation associated with arthritis (Scorei et. al., Biological Trace Element Research 144, no. 1-3 (December 2011): 253-63). CF has also been reported for use in the treatment of skin (US 6080425) and in attempts to reduce the rate of hair growth (US 5985842).

Synthesis of CF has been described in various sources, and one exemplary protocol for small scale synthesis (10ml) can be found in US 6924269 in which 0.62 g boric acid was reacted with 3.60 g fructose in 10 ml of water, with subsequent neutralization using 1 g calcium carbonate under evolution of carbon dioxide. A substantially similar process at a very small scale (4ml) is described in WO 98/43652.

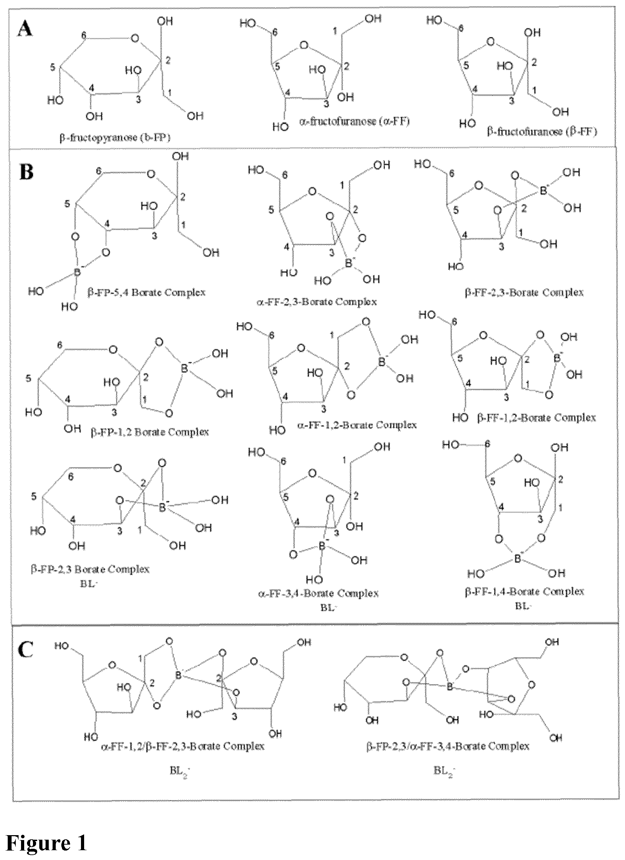

While such process is at least conceptually simple on paper, it should be recognized that there is substantial complexity involved upon closer investigation. At the outset, commercially available fructose exists in numerous isomeric forms, having five-membered heterocyclic rings (furanose) and six-membered (pyranose) heterocyclic rings, each with their own respective stereoisomeric configuration at the anomeric carbon atom, leading to respective alpha and beta forms. Still further, fructose may also exist in open-chain forms. To complicate matters, the boric acid molecule forms diester complex bonds with two hydroxyl groups of a sugar molecule. As fructose has five hydroxyl groups (several of them in vicinal position), numerous ester products can be formed with each of the stereoisomeric form of fructose. In addition, due to the remaining hydroxyl groups in the boric acid after esterification with a first sugar molecule, further diester complex bonds can be formed with a second sugar molecule, at various positions. Exemplary stereoisomers for fructose are shown in Panel A of **Figure 1**, while exemplary mono-complexes are depicted in Panel B of Figure 1and exemplary di-complexes are depicted in Panel C of Figure 1. Thus, and not surprisingly, only very little information on reaction dynamics and specific product formation is known for boro-carbohydrate complexes. Not surprisingly, neither US 6924269 nor WO 98/43652 make any reference to any ratios of di- to mono-complexes, or make reference to residual boric acid in the system.

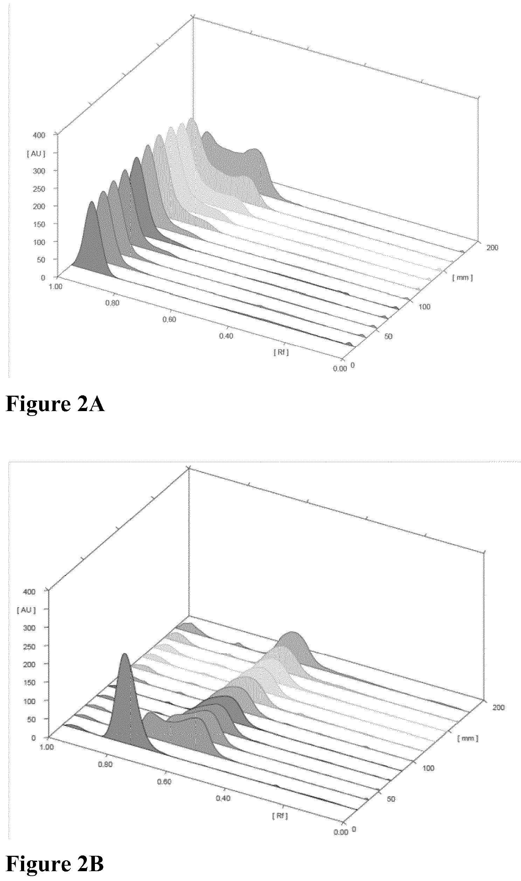

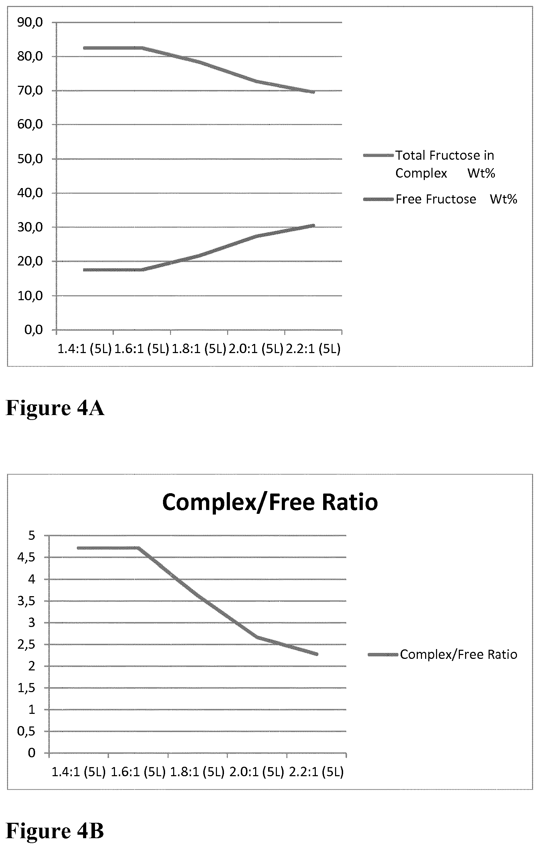

For example, Edwards et al. (Journal of Food Research 3, no. 3 (May 15, 2014)) report an NMR analysis of fructoborate complexes and their distribution of stereoisomers along with stability data, and Makkee et. al. (Recueil Des Travaux Chimiques Des Pays-Bas 104, no. 9 (September 2, 2010): 230-35), being the closest piece of prior art, describe selected processes for preparation of borate complexes with saccharides in small scale under selected reaction conditions in an attempt to characterize formation of various forms. However, all or almost all of the conditions that were described as providing CF as a di-complex suffered from very low yields and/or substantial residual quantities of boric acid, which is generally undesirable. For example, as can be seen from the graphs in Figs. 2 and 4, relatively high ratios of di- to mono-complexes with relatively low quantities of boric acid were present at a pH 6-12 at small scale reaction volumes (5 ml) and under conditions of 5- and 10-fold molar excess of sugar to boric acid (4^{th} graph: 0.5M sugar, 0.1M boric acid; 5^{th} graph: 1.0M sugar 0.1M boric acid). As can be readily taken from the concentrations reported under these reaction conditions (note: y-axis maximum is 0.1M), is 20 mol% at best for the overall yield of complexes and 18 mol% for the di-complex at best. Reduction of molar excess under the conditions reported by Makkee led to substantial quantities of free boric acid and even lower yields of the complexes. Residual boric acid is very undesirable due to its potential toxicity and other possible interference with biological molecules (*e.g*., boric acids are known to act as inhibitor to certain enzymes (*e.g*., urease) or Rho family of GTP-binding proteins).

Thus, while CF and other carbohydrate complexes are well known in the art, there is still a need for a process that results in a high-yield of di-complex calcium fructoborate or other boro-carbohydrate complexes. Viewed from a different perspective, it would be desirable to have a process that provides a composition comprising calcium fructoborate or other boro-carbohydrate complex with low (*e.g.,* ≤ 10 wt%) residual free boric acid in the product. In the same way, it would be desirable to have a process that provides a composition with calcium fructoborate or another boro-carbohydrate complex without large amounts (*e.g*., ≥ 30 wt%) of residual fructose or other carbohydrate in the product. Finally, and viewed from yet another perspective, it would be desirable to have a process that provides a composition with a di-complex to free boric acid ratio that is at least 10:1, more preferably at least 15:1, and most preferably at least 20:1 as it is thought that the di-complex is the biologically most relevant and therefore most desirable form of CF.

### Summary of The Invention

The present invention is defined by the claims. Any "embodiment", "aspect" or "example" which is disclosed in the description but is not covered by the claims should be considered as presented for illustrative purpose only.

The present invention relates to a method of increasing a ratio of di-complex content to unreacted boric acid in a preparative reaction for producing fructoborate complexes, wherein said complexes are prepared by a method comprising:
selecting a molar ratio between a fructose and boric acid of between 1.8:1 and 2.4:1;
reacting the fructose and boric acid in an aqueous solution at the molar ratio to generate a liquid composition comprising fructoborate di-complexes and borate, wherein the preparative scale for the reaction is at least 1000 ml; and
drying the liquid composition to generate a dry product comprising fructoborate dicomplexes and borate.

Also disclosed is a method of producing a composition that comprises fructoborate complexes in an amount of at least 65 wt%, wherein the fructoborate complexes include di-complexes and mono-complexes, wherein the composition further comprises boric acid, and wherein the di-complex and the boric acid are present in a ratio of at least 10:1, the method comprising:
reacting fructose with boric acid in water, and neutralizing the reaction with at least one of an alkaline metal hydroxide, an earth alkaline metal hydroxide, an alkaline metal carbonate, and an earth alkaline metal carbonate to provide a cation to form a salt of the fructose complexes;
selecting a molar ratio between the fructose and the boric acid of at least 1.8:1;
selecting a preparative scale for the reaction of at least 1000 ml; and
reacting the fructose with the boric acid at the ratio and the scale to thereby form the fructose complexes, and wherein the cation forms the salt of the fructose complexes.

Preferably the step of reacting forms the fructose complexes in an amount of at least 70 wt%.

Preferably the molar ratio between the fructose and the boric acid is between 1.8:1 and 2.4:1.

Preferably the preparative scale for the reaction is at least 5,000 ml.

Preferably the di-complex and the boric acid are present in a ratio of at least 15:1.

Preferably the di-complex and the boric acid are present in a ratio of at least 20:1.

Preferably the composition is a liquid composition.

Preferably the cation is a calcium cation or a magnesium cation.

Preferably the composition has a pH of less than 6.0.

Further disclosed is a liquid composition comprising:
a boron-containing fraction comprising a plurality of fructoborate complexes and boric acid;
wherein the fructoborate complexes are a mixture of a di-complex and a mono-complex; and
wherein the di-complex is present in the boron-containing fraction of the composition in an amount of at least 75 wt%, wherein the boric acid constitutes less than 13 wt% of the boron-containing fraction of the composition, and wherein a ratio of fructoborate complexes to unreacted fructose is at least 1.3 to 1.

Preferably a ratio of the di-complex to the mono-complex in the mixture is between 10:1 and 12:1.

Preferably the di-complex is present in the composition in an amount of at least 80 wt%.

Preferably the di-complex is present in the composition in an amount of at least 85 wt%.

Preferably unreacted boric acid is present in the composition in an amount of less than 10 wt%.

Preferably unreacted boric acid is present in the composition in an amount of less than 5.0 wt%.

Disclosed are various borocarbohydrate complex-containing compositions and methods of their production. In especially noteworthy aspects of the inventive subject matter, the borocarbohydrate complex-containing compositions have a very high content of di-complex, a very low content of unreacted boric acid, and are obtained at remarkably high yields.

Disclosed is a method of producing a composition that comprises borocarbohydrate complexes in an amount of at least 65 wt%, wherein the borocarbohydrate complexes include di-complexes and mono-complexes, wherein the composition further comprises boric acid, and wherein the di-complex and the boric acid are present in a ratio of at least 10:1. Particularly contemplated methods include a step of selecting a molar ratio between a carbohydrate and the boric acid of at least 1.8:1, and a further step of selecting a preparative scale for the reaction of at least 1000 ml. The carbohydrate is then reacted with the boric acid at the ratio and the scale to thereby form the carbohydrate complexes. In most embodiments, a cation is added to form a salt of the carbohydrate complexes.

In some aspects, the step of reacting forms the carbohydrate complexes in an amount of 70 wt%, and/or the molar ratio between the carbohydrate and the boric acid is between 1.8:1 and 2.4:1, and/or the preparative scale for the reaction is at least 5,000 ml. Therefore, it is also contemplated that the di-complex and the boric acid are present in a ratio of at least 15:1, or in a ratio of at least 20:1. Most typically, the composition is a liquid composition, and/or the carbohydrate is fructose, and/or the cation is a calcium cation or a magnesium cation. It is further contemplated that the composition has a pH of less than 6.0.

Further disclosed is a method of producing a composition comprising borocarbohydrate complexes having a borocarbohydrate di-complex to boric acid ratio of at least 5:1. In such methods, a molar ratio between the carbohydrate and the boric acid of at least 1.6:1 is selected and a preparative scale for the reaction of at least 200 ml is selected. The carbohydrate is then reacted with the boric acid and a compound that comprises a cation at an acidic pH and at the ratio and the scale to so form the carbohydrate complexes, wherein the carbohydrate complexes form a salt with the cation.

The molar ratio between the carbohydrate and the boric acid is between 1.8:1 and 2.4:1, and the preparative scale for the reaction is at least 1,000 ml. In most aspects, the acidic pH is a pH of less than 6.0. Most typically, the borocarbohydrate complexes are present in the composition in an amount of at least 60 wt%, and/or the borocarbohydrate di-complex to boric acid ratio is at least 10:1. The compound that comprises a cation is in many embodiments an alkaline metal hydroxide, an earth alkaline metal hydroxide, an alkaline metal carbonate, or an earth alkaline metal carbonate, and/or the carbohydrate is fructose.

Therefore, disclosed is also a method of increasing di-complex content in a preparative reaction having a first reaction scale to form a composition comprising at least 65 wt% borocarbohydrate complexes, wherein the borocarbohydrate complexes are a mixture of di-complexes and mono-complexes. Such methods will typically include a step of selecting a molar ratio between the carbohydrate and the boric acid of at least 1.4:1, and a further step of increasing the first reaction scale (*e.g.,* at least 200 ml) to a second reaction scale, and reacting the carbohydrate and the boric acid at the ratio at the second reaction scale (at least 1,000 ml) to thereby increase the di-complex content in the second reaction scale as compared to the first reaction scale.

The carbohydrate is fructose, and the step of increasing the first reaction scale to the second reaction scale also decreases unreacted boric acid in the second reaction scale as compared to the first reaction scale. The molar ratio between the carbohydrate and the boric acid is between 1.8:1 and 2.4:1. Water is removed from the composition (*e.g*., via freeze-drying or spray drying).

Thus, viewed from a different perspective, and in a method of producing a composition comprising borocarbohydrate complexes and boric acid, wherein the borocarbohydrate complexes are a mixture of di-complexes and a mono-complexes, an improvement may comprise a step of reacting a carbohydrate with boric acid at an acidic pH and at a molar ratio between the carbohydrate and the boric acid of at least 1.8:1, wherein the step of reacting is performed at a preparative scale of at least 1000 ml to thereby achieve a ratio of di-complex to residual boric acid of at least 10:1.

The carbohydrate is fructose, and, most typically, the pH is less than 6.0. The molar ratio between the carbohydrate and the boric acid is between 1.8:1 and 2.4:1. Therefore, the ratio of di-complex to residual boric acid of at least 15:1 or at least 20:1. As before, it is contemplated that such improvements may include a step of removing water from the composition.

Disclosed is a method of producing a composition with a boric acid content of equal or less than 15 wt% is contemplated, wherein the composition comprises at least 70 wt% borocarbohydrate complexes, and wherein the borocarbohydrate complexes are a mixture of di-complexes and mono-complexes. Such methods will include a step of selecting a molar ratio between the carbohydrate and the boric acid such that the carbohydrate is in molar excess over the boric acid, and a further step of selecting a preparative scale for the reaction of at least 1000 ml, and a still further step of reacting the carbohydrate and the boric acid at an acidic pH to thereby produce the composition with the boric acid content of equal or less than 15 wt%.

In such methods, it is contemplated that the boric acid content of the composition is equal or less than 10 wt% or equal or less than 5 wt%, and/or that the molar ratio between the carbohydrate and the boric acid is between 1.6:1 and 2.2:1, and/or that the acidic pH is a pH of less than 6.0.

Also disclosed is a liquid composition that comprises a plurality of borocarbohydrate complexes and boric acid, wherein the borocarbohydrate complexes are a mixture of a di-complex and a mono-complex, and wherein the di-complex is present in the composition in an amount of at least 75 wt% and wherein the boric acid constitutes less than 13 wt% of the composition.

Most typically, the ratio of the di-complex to the mono-complex in the mixture is between 10:1 and 12:1, and/or the di-complex is present in the composition in an amount of at least 80 wt% or at least 85 wt%, while unreacted boric acid is present in the composition in an amount of less than 10 wt%, or less than 5.0 wt%.

Further disclosed is a liquid composition having an acidic pH and comprising a borocarbohydrate di-complex and boric acid, wherein the borocarbohydrate di-complex and the boric acid are present in a ratio of at least 10:1. The acidic pH in such compositions is a pH of less than 6.0, and/or the borocarbohydrate di-complex and the boric acid are present in a ratio of at least 15:1, or at least 20:1, while the unreacted boric acid is present in an amount of less than 10 wt% or less than 5 wt%. It is further contemplated that in such compositions the borocarbohydrate di-complex is present in an amount of at least 80 wt%.

Also disclosed is a liquid composition comprising a borocarbohydrate di-complex, a borocarbohydrate mono-complex and boric acid, wherein a ratio between the borocarbohydrate di-complex and the borocarbohydrate mono-complex is at least 10:1, and wherein the boric acid is present in the composition in an amount of equal or less than 10 wt%.

The ratio between the borocarbohydrate di-complex and the borocarbohydrate mono-complex in such compositions is at least 15:1, or at least 20:1, and/or the ratio between the borocarbohydrate di-complex and the borocarbohydrate mono-complex is at least 25:1, while in further aspects the boric acid is present in the composition in an amount of equal or less than 7.5 wt%, or less than 5.0 wt%. Most typically, the liquid composition has a pH of less than 6.0.

Disclosed is a liquid reaction mixture having an acidic pH comprising a carbohydrate, boric acid, and borocarbohydrate complexes, wherein the borocarbohydrate complexes are a mixture of di-complexes and mono-complexes, and wherein the di-complexes and the boric acid are present in a ratio of at least 5:1, and wherein a ratio of the borocarbohydrate complexes to the carbohydrate is between 1.5 and 4.5. Most typically, the ratio of the borocarbohydrate complexes to the carbohydrate is between 2.0 and 3.5, and/or the pH is less than 6.0 while the liquid reaction mixture has a volume of at least 200 ml.

Also disclosed is a composition comprising a borocarbohydrate di-complex and unreacted boric acid, wherein the borocarbohydrate di-complex and the boric acid are present in the composition in a ratio of at least 10:1 in liquid form and at least 20:1 in dried form. In such compositions, the borocarbohydrate di-complex and the boric acid are present in the composition at a ratio of at least 15:1 in liquid form and at least 22:1 in dried form, and/or the boric acid is present in the composition in an amount of less than 10 wt%, or less than 5.0 wt%.

In view of the above, a composition is disclosed comprising a borocarbohydrate complex that is produced by a process having the steps of (a) selecting a molar ratio between the carbohydrate and the boric acid of at least 1.6:1; (b) selecting a preparative scale for the reaction of at least 1000 ml; and (c) reacting the carbohydrate and the boric acid at an acidic pH to thereby obtain a composition that comprises a borocarbohydrate di-complex and boric acid at a ratio of at least 5:1.

Most typically, the molar ratio between the carbohydrate and the boric acid is between 1.8:1 and 2.2:1, and/or the acidic pH is a pH of less than 6.0, and/or the composition comprises the borocarbohydrate di-complex and the boric acid at a ratio of at least 10:1 or at least 15:1.

Various objects, features, aspects and advantages of the inventive subject matter will become more apparent from the following detailed description of preferred embodiments, along with the accompanying drawing figures in which like numerals represent like components.

### Brief Description of the Drawing

Figure 1 shows exemplary stereoisomeric forms of fructose (Panel A), fructoborate mono-complexes (Panel B), and fructoborate di-complexes (Panel C).
Figures 2A and 2B show graphs for densitometry readings of TLC tracks. Tracks in Figure 2A represent individual reactions of fructose and boric acid with molar ratios ranging from 1:10 to 1:1, and tracks in Figure 2B represent individual reactions of fructose and boric acid with molar ratios ranging from 1:1 to 10:1.

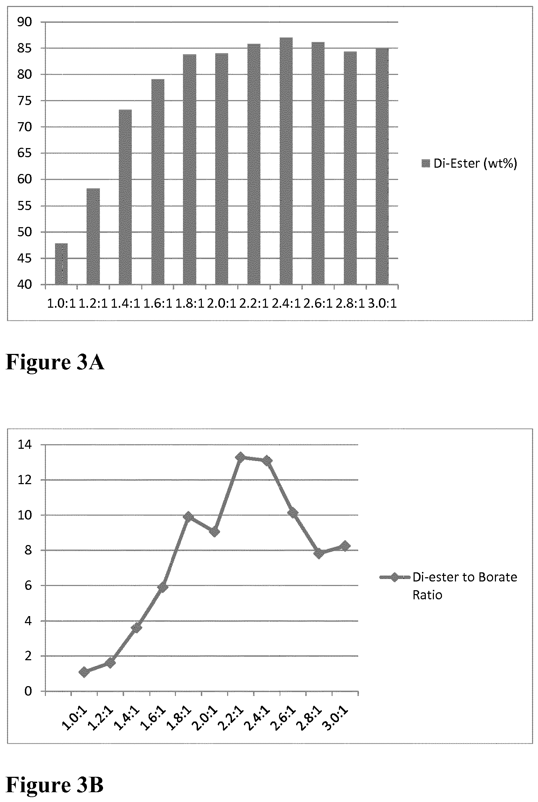

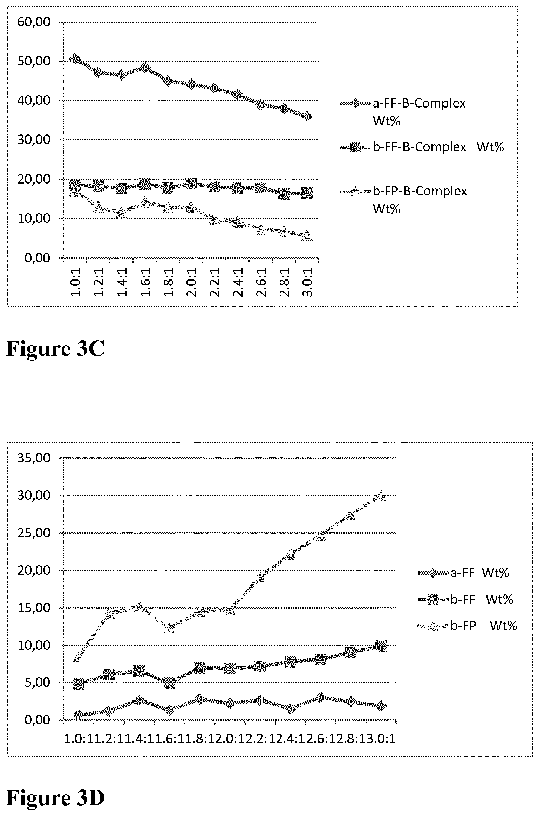

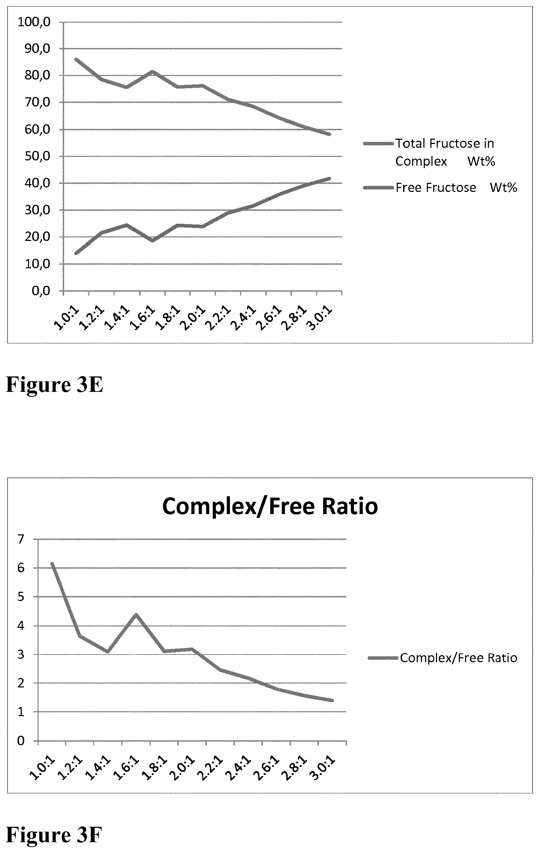

Figures 3A to 3F illustrate exemplary results for selected product parameters at a 200 ml scale and molar ratios of fructose to boric acid between 1:1 and 3:1. Figure 3A lists di-complex ('di-ester') formation as a function of molar ratio; Figure 3B shows the ratio of di-complex to unreacted boric acid as a function of molar ratio; Figure 3C provides compositional information about stereochemical aspects in fructoborate complexes as a function of molar ratio; Figure 3D is a line graph showing corresponding compositional information about stereochemical aspects in unreacted fructose as a function of molar ratio; Figure 3E illustrates yields of total fructose complexes (mono- and di-complexes) relative to unreacted fructose as a function of molar ratio; Figure 3F is a line graph illustrating the ratio of total fructose complexes to unreacted fructose as a function of molar ratio.
Figures 4A to 4B illustrate exemplary results for selected product parameters at a 5,000 ml scale and molar ratios of fructose to boric acid between 1.4:1 and 2.2:1. Figure 4A illustrates yields of total fructose complexes (mono- and di-complexes) relative to unreacted fructose as a function of molar ratio; Figure 4B is a line graph illustrating the ratio of total fructose complexes to unreacted fructose as a function of molar ratio.

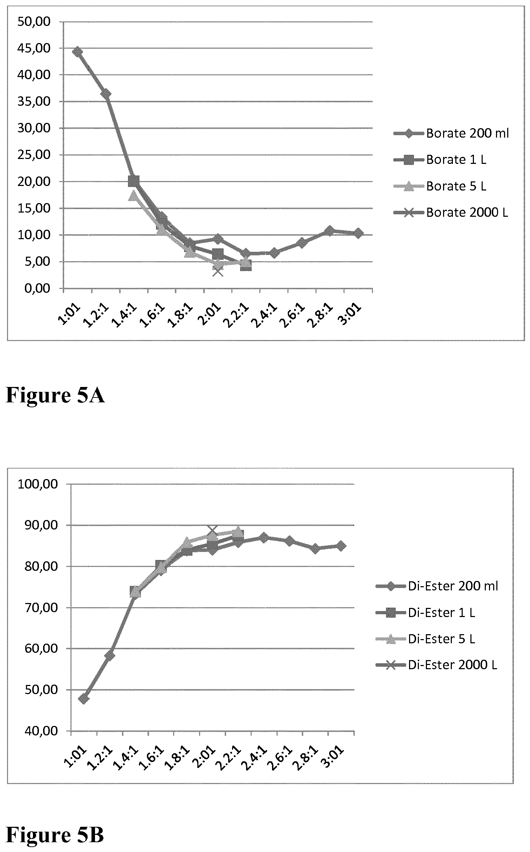

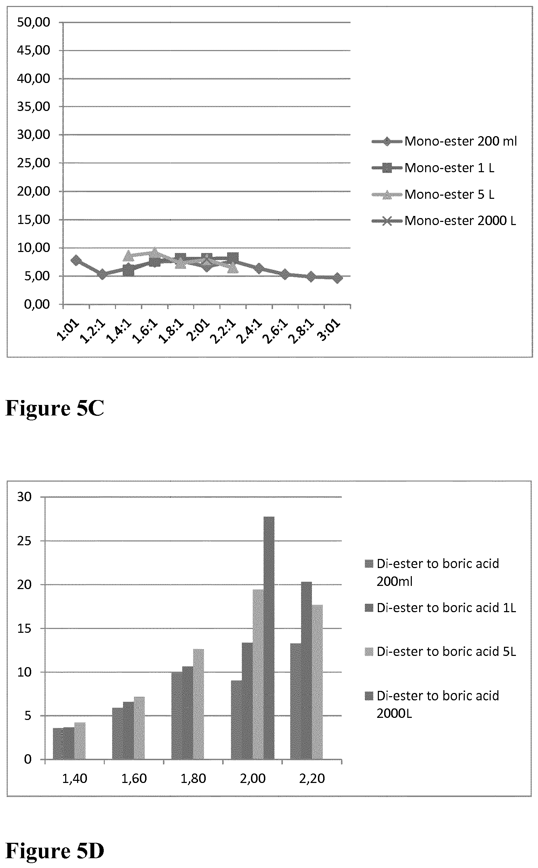

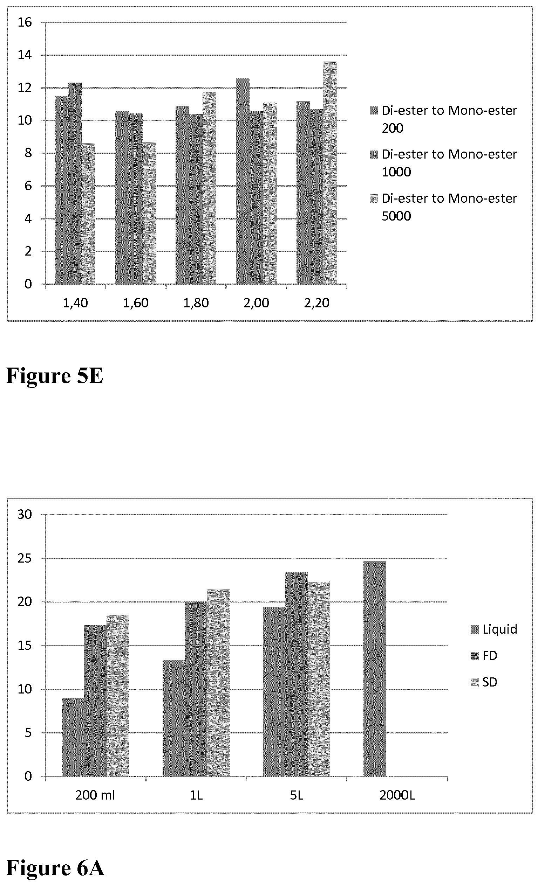

Figures 5A to 5E depict exemplary results for selected product parameters at escalating production scales and variable molar ratios of fructose to boric acid. Figure 5A is a line graph showing a decrease of free (unreacted) boric acid as a result of scale up at the same molar ratios over a range of preset molar ratios. Figure 5B is a line graph showing an increase of di-complex as a result of scale up at the same molar ratios over a range of preset molar ratios. Figure 5C is a line graph showing that the quantities of mono-complex is substantially unaffected by scale up at the same molar ratios over a range of preset molar ratios. Figure 5D is a bar graph showing an increase of the ratio of di-complex to free boric acid as a result of scale up and an exacerbated increase of the same ratio as a function of molar ratios between fructose and boric acid. Figure 5E is a bar graph indicating that the ratio of di-complex to mono-ester is substantially unaffected by scale up and molar ratios between fructose and boric acid.

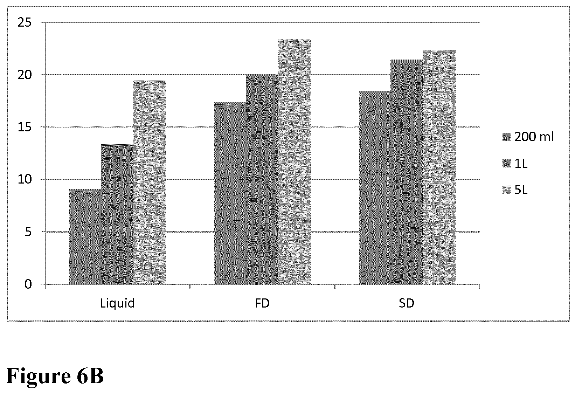

Figures 6A to 6B show exemplary results for selected product parameters of reconstituted compositions after water had been removed using a fixed molar ratio between fructose and boric acid. Figure 6A shows ratios of di-complex to free boric acid in liquid, reconstituted freeze-dried (FD), and reconstituted spray-dried (SD) forms as a function of production scale. Figure 6B shows ratios of di-complex to free boric acid in various production scales as a function of state (liquid, reconstituted freeze-dried (FD), and reconstituted spray-dried (SD)).

### Detailed Description

The inventors have discovered various methods and conditions that allow production of borocarbohydrate complex-containing compositions with high content of di-complexes, very low content of unreacted boric acid, and low quantities of unreacted fructose, all at remarkably high yields.

For example, as described in more detail below, total carbohydrate complexes could be produced at yields of ≥ 60 wt%, >_ 65 wt%, >_ 70 wt%, or ≥ 75 wt%. As is also described in more detail below, unreacted boric acid was limited to quantities of ≤ 10 wt%, ≤ 8 wt%, ≤ 6 wt%, or ≤ 4 wt%. As described in more detail below, the yields of the di-ester were remarkably high, for example, at a di-ester to mono-ester ratio of at least 8:1, at least 9:1, at least 10:1, or at least 11:1, and/or at a di-ester to unreacted boric acid of at least 9:1, at least 10:1, at least 15:1, at least 20:1, or at least 25:1 as also shown in more detail below. Therefore, total carbohydrate complexes can be produced with low residual quantities of carbohydrate, at for example, ≤ 35 wt%, ≤ 30 wt%, ≤ 25 wt%, or ≤ 20 wt% unreacted carbohydrate. Unless specified otherwise, all percentages are indicated as wt% of the total of all reaction products and unreacted reagents.

Despite the seemingly simple reaction of boric acid and carbohydrates to form esters, the inventors discovered that numerous reaction parameters have unexpected and significant impact on various aspects of product formation, and especially on quantities of di-complex, overall yield of borocarbohydrate complexes, and residual (unreacted) boric acid. For example, while a molar excess of boric acid over fructose generally increases overall complex formation, inverting these molar proportions (e.g., using slight molar excess of fructose over boric acid) lead to an optimum area of di-complex formation with apparent saturation starting at a ratio of about 1.8. Even more unexpectedly, increasing the molar ratio of fructose to boric acid (between 1:1 and 3:1) revealed that at a ratio of about 2.2-2.4:1 free boric acid was at a minimum, but that there was a balance point in maximizing yield of complex formation (where a lower fructose to boric acid ratio was better) and free boric acid (where a higher fructose to boric acid ratio was better). Additionally, the inventors unexpectedly discovered that a simple scale-up of the reaction at particular molar ratios of carbohydrate to boric acid ratio using otherwise identical process conditions decreased unreacted boric acid and also increased the yield of the di-complex (especially at molar ratios of carbohydrate to boric acid ratio of 1.4:1 to 2.2:1). Oddly, the formation of mono-complexes was substantially unaffected.

In another unexpected finding on scale-up, the inventors also discovered that the ratio of di-complex to free boric acid was almost entirely insensitive to scale-up where the molar ratios of fructose to boric acid was below 1.6:1 (*e.g.,* 1.4:1 to 1.6:1), was only slightly affected at a molar ratio of fructose to boric acid of 1.8:1, and was substantially affected (*e.g*., almost linear at 2.0:1) with an apparent optimum 5L scale for 2.2:1 as also shown in more detail below. The boron in the borocarbohydrate complexes is provided in the form of boric acid, and most typically in the form of an aqueous solution.

In many embodiments, the preparation of the borocarbohydrate complexes is based on reacting fructose in solution with boric acid for a time sufficient to allow the reaction to run to completion and/or the reaction mixture to become clear (*e.g.,* at least 30 min, at least 60 min, or at least 90 min). After the reaction is completed, the borocarbohydrate complexes can be charge neutralized with a cation-containing compound, which is preferably an alkaline metal hydroxide, an earth alkaline metal hydroxide, an alkaline metal carbonate, and/or an earth alkaline metal carbonate (*e.*g., calcium carbonate). Most typically, the cation-containing compound is added in an amount that is between an equimolar amount (with respect to boric acid) and 10% of the molar amount of boric acid, most typically between 0.7 and 0.3 (*e.g.,* 0.5) times the molar amount of boric acid. Where the cation-containing compound is a hydroxide or carbonate, it is in most aspects preferred that the cation-containing compound is added slowly (*e.g*., over a period of at least 10 min, at least 20 min, or at least 30 min), and where appropriate in multiple batches (*e.g.,* at least two, three or four, etc.). The so prepared liquid composition can then be further combined with other components, or compounded with one or more beverages. Alternatively, water can be at least partially removed to obtain a concentrate or dry product (*e.g*., via freeze drying or spray drying) that can then be used as or compounded with a nutritional supplement.

### Example Reaction:

The reaction according to scheme 1 below was performed at a 5L scale and a molar ratio between fructose and boric acid of 2:1. First, fructose was dissolved in water at a temperature of 20-25 °C. Solid boric acid was then added at a molar ratio of 2:1, and the mixture was allowed to react under continuous stirring for 90 minutes at a temperature of 20-25 °C. CaCO3 was added in an amount that is 50% of the molarity of the boric acid over a period of 30 minutes in three equal batches under continuous stirring. The pH of the reaction was typically neutral to moderately acidic (pH of less than 6.0).

C₆H₁₂O₆ (frc) + H₃BO₃ (B) + CaCO₃ --> [frc-B-frc]⁻₂ Ca²⁺ + [frc-B]⁻₂ Ca²⁺ + CO₂ + H₂O (Scheme 1)

### Experiments and Results

Unless indicated otherwise, all reactions were performed at the indicated scale and molar ratios between fructose and boric acid by first dissolving fructose in water at a temperature of 20-25 °C. Solid boric acid is then added at the selected molar ratio, and the mixture is reacted under continuous stirring for 90 minutes at a temperature of 20-25 °C. Where desired, CaCO₃ is then added in an amount that is 50% of the molarity of the boric acid over a period of 30 minutes in three equal batches under continuous stirring (*e.g*., where 1 mol of boric acid was used, 0.5 mol CaCO₃ was used). The pH of the reaction was typically neutral to moderately acidic, and in most cases at a pH of less than 6.0.

Liquid-state ¹¹B, ¹³C, and ¹H NMR was performed on a Varian Mercury 300MVX NMR spectrometer equipped with a 5mm Varian ATB Probe at resonance frequencies of 96.14 MHz (¹¹B), 75.36 MHz (¹³C) and 299.67 MHz (¹H), respectively. ¹¹B spectra were acquired with a 45 degree tip angle pulse width, a relaxation delay of 0.2 seconds, an acquisition time of 80 ms with 8K points acquired with a spectral width of 100 kHz, and 1024 pulses were averaged. The data was zero filled to 65K points. The ¹³C NMR was acquired with a 30 degree tip angle pulse width, a relaxation delay of 5 seconds, 0.96 second acquisition time, with 24K points acquired with a spectral width of 25 kHz, and 10-12,000 pulses were averaged. The data was zero filled to 131K points. The ¹H NMR spectra were obtained with a 30 degree pulse angle, a relaxation delay of 2 seconds, a 4.448 second acquisition time, with 32K points acquired over a spectral width of 7.2 kHz, and 128 pulses were averaged. The data was zero-filled to 131K points. The data was acquired in a quantitative manner with inverse gated decoupling of protons during the acquisition of the ¹¹B and ¹³C experiments. All samples were dissolved in D₂O (Cambridge Isotope Laboratories). No pH adjustments were performed on the samples after dissolution.

Solid-State ¹³C (50.30 MHz), ¹¹B (64.17 MHz) NMR spectra were obtained on a Varian UnityPlus-200 NMR spectrometer equipped with a Doty Scientific 7 mm Supersonic CP-MAS probe. Magic angle spinning (MAS) speeds of around 6 kHz were employed. The ¹³C NMR data was acquired using cross polarization which prepares the magnetization on the protons initially and then transfers the spin locked magnetization to the ¹³C nuclei. The advantage of this experiment is the fact that it is performed at the spin-lattice relaxation rate (T1) of protons in the sample which is considerably shorter than the T1 of ¹³C nuclei in the same sample. Thus, one obtains a significant enhancement of the ¹³C signal from the polarization transfer and can pulse at a shorter pulse-repetition rate. The ¹³C CP-MAS experiment on calcium fructoborate complex was performed with a 1ms variable amplitude contact time, an 8 second relaxation rate, and an acquisition time of 25.6 ms, with 1K points being acquired over a spectral with of 40 kHz, and 4096 pulses were averaged. The exceptions to these acquisition parameters were those used for pure crystalline fructose. The ¹¹B NMR spectra were acquired with MAS and with the sample remaining static in the NMR probe. The experiments were acquired with a central transition selective pulse width, a 0.2 second relaxation time, with 1K points being acquired in an acquisition time of 10.2 ms, and with a spectral width of 100 kHz.

Samples were observed directly after they were received, after they had been thermally treated in a Duratech TCON dry bath system (capable of holding temperatures to +/- 0.1 °C), or as calibration standards which were made by mixing accurately weighed samples of calcium fructoborate with magnesium stearate or maltodextrin. Samples for solid-state NMR were weighed to the nearest 0.1 mg on a Sartorius GD-503-NTEP microbalance after they were packed into the MAS rotor.

Initial studies had shown that overall borocarbohydrate complex formation was favored under substantial molar excess of boric acid relative to fructose as can be seen from Figures 2A and 2B. More particularly, **Figure 2A** illustrates optical densitometry readings from a TLC plate onto which were spotted aliquots of small scale reaction volumes (*e.g*., 2 ml) with decreasing molar ratios between fructose and boric acid. Reading the lanes from front to back, the first lane is fructose control, while the following next 10 lanes reflect a decrease starting at 10:1 (fructose to boric acid) to 1:1 (fructose to boric acid). As is readily evident, appreciable quantities of total complex formation begin to develop at equimolar ratios (see last lane). Similarly, **Figure 2B** illustrates optical densitometry readings from a TLC plate onto which were spotted aliquots of small scale reaction volumes (*e.g*., 2 ml) with increasing molar ratios between boric acid and fructose. Again, reading the lanes from front to back, the first lane is fructose control, while the following next 10 lanes reflect an increase starting at 1:1 (fructose to boric acid) to 1:10 (fructose to boric acid). As can be seen, appreciable total complex formation is favored at a molar ratio of 1:2 (fructose to boric acid), and is nearly quantitative at a molar ration of 1:5 (fructose to boric acid). Thus, under the conditions observed, molar excess of boric acid drove the formation of total borocarbohydrate complex yield.

In an effort to further investigate the reaction conditions and product compositions, the inventors performed numerous experiments at various production scales and various molar ratios between the carbohydrate (*e.g*., fructose) and boric acid. Quite unexpectedly, the inventors found that the specific product composition is substantially affected by at least the molar ratios between the carbohydrate (*e.g*., fructose) and boric acid and/or the production scale. Contrary to the skilled artisan's approach of using a high boric acid to carbohydrate ratio to so increase overall yield of the borocarbohydrate complex as suggested by the data in Figures 2A and 2B, the inventors discovered that by selection of the appropriate molar ratio between the carbohydrate and the boric acid an enhanced yield of di-complex can be obtained at concurrent low quantities of unreacted boric acid.

For example, the inventors modified the molar ratio between the carbohydrate (here: fructose) and boric acid over a relatively large range from an equimolar ratio to a 3:1 molar excess of fructose to boric acid at a 200 ml production scale. Notably, based on quantitative ¹¹B NMR analysis and as can be clearly seen in **Table 1** below, quantities of unreacted boric acid significantly decreased with increasing molar ratio (in the range from 1.0:1 to 2.4:1), and then moderately rose with further increasing molar ratio (in the range from 2.6:1 to 3.0:1. Thus, it should be appreciated that an increase in the molar ratio between the carbohydrate (here: fructose) and boric acid over a range of at least 1.8:1 to 2.6:1 had the unexpected technical effect of decreasing quantities of unreacted boric acid. Conversely, the quantities of the di-complex increased as a function of an increasing molar ratio between the carbohydrate (here: fructose) and boric acid over a relatively large range from an equimolar ratio to a 3:1 molar excess of fructose to boric acid at a 200 ml production scale, possibly with a saturation effect starting at a molar ratio of about 1.8:1. Thus, compositions with especially high di-complex content could be obtained at molar ratios at and above 1.6:1 or 1.8:1. Therefore, an increase in the molar ratio between the carbohydrate (here: fructose) and boric acid starting at 1.6:1 or 1.8:1 had the unexpected technical effect of increasing quantities of di-ester. Remarkably, the increase in molar ratio between fructose and boric acid had little to no effect on the quantities of mono-ester produced by the reaction.

**Figure 3A** exemplarily illustrates the dramatic increase in di-ester yield as a function of increasing molar ratio between the carbohydrate (here: fructose) and boric acid. The ratio of di-ester to unreacted boric acid is exemplarily depicted in **Figure 3B****,** where the optimum range for the ratio is between 1.8:1 and 2.6:1.

**Table 1 (not according to the invention)**

| **Molar Ratio** | **Borate (wt%)** | **Di-Ester (wt%)** | **Mono-ester (wt%)** | **Di-ester/Borate Ratio** |
|---|---|---|---|---|
| *Volume 200 ml* | | | | |
| **1.0:1** | 44.34 | 47.84 | 7.81 | 1.08 |
| **1.2:1** | 36.42 | 58.26 | 5.32 | 1.60 |
| **1.4:1** | 20.32 | 73.28 | 6.40 | 3.61 |
| **1.6:1** | 13.38 | 79.12 | 7.50 | 5.91 |
| **1.8:1** | 8.46 | 83.84 | 7.70 | 9.91 |
| **2.0:1** | 9.28 | 84.04 | 6.68 | 9.05 |
| **2.2:1** | 6.46 | 85.86 | 7.67 | 13.28 |
| **2.4:1** | 6.64 | 87.01 | 6.35 | 13.10 |
| **2.6:1** | 8.50 | 86.19 | 5.31 | 10.14 |
| **2.8:1** | 10.78 | 84.33 | 4.89 | 7.82 |
| **3.0:1** | 10.30 | 85.02 | 4.68 | 8.25 |

Remarkably, ¹³C NMR analysis also revealed that an increase in the molar ratio of carbohydrate (here: fructose) to boric acid decreased total complex (di- plus mono-complex) formation most significantly for the alpha-fructofuranose form, moderately for the beta-fructofuranose form, and negligibly or not for the beta-fructopyranose form as can be taken from **Table 2** below. Also, the most pronounced decrease in total complex forms for the alpha-fructofuranose form and the beta-fructofuranose form started at a ratio of 1.6:1 or 1.8:1, which appears to be a counter-trend to the specific increase in di-ester formation at comparable ratios. Thus, an increase of the molar ratio of carbohydrate (here: fructose) to boric acid above 1.6:1 or 1.8:1 had the surprising technical effect of decreasing total complexes in alpha-fructofuranose form and to some extent beta-fructofuranose form. Figure 3C is a graphic representation of the results of Table 2.

**Table 2 (not according to the invention)**

| **Molar Ratio** | **a-FF-B-Complex (wt%)** | **b-FF-B-Complex (wt%)** | **b-FP-B-Complex (wt%)** |
|---|---|---|---|
| *Volume 200 ml* | | | |
| **1.0:1** | 50.58 | 18.42 | 17.02 |
| **1.2:1** | 47.15 | 18.31 | 13.02 |
| **1.4:1** | 46.44 | 17.68 | 11.47 |
| **1.6:1** | 48.44 | 18.79 | 14.20 |
| **1.8:1** | 45.00 | 17.79 | 12.89 |
| **2.0:1** | 44.19 | 18.93 | 13.02 |
| **2.2:1** | 43.00 | 18.10 | 9.97 |
| **2.4:1** | 41.60 | 17.75 | 9.12 |
| **2.6:1** | 38.97 | 17.88 | 7.34 |
| **2.8:1** | 37.93 | 16.21 | 6.80 |
| **3.0:1** | 36.05 | 16.49 | 5.70 |

Likewise, ¹³C NMR analysis revealed that an increase in the molar ratio of carbohydrate (here: fructose) to boric acid increased unreacted carbohydrate (here:fructose) most significantly for the beta-fructopyranose form, moderately for the beta-fructofuranose form, and negligibly or not for the alpha-fructofuranose form as can be taken from **Table 3** below. Once more, the most pronounced increase in unreacted carbohydrate for the beta-fructopyranose form and the beta-fructofuranose form started at a ratio of 1.6:1 or 1.8:1. Therefore, an increase of the molar ratio of carbohydrate (here: fructose) to boric acid above 1.6:1 or 1.8:1 had the unexpected technical effect of the unreacted carbohydrate for the beta-fructopyranose and the beta-fructofuranose forms as is also shown in **Figure 3D****.**

**Table 3 (not according to the invention)**

| **Molar Ratio** | **a-FF (wt%)** | **b-FF (wt%)** | **b-FP (wt%)** |
|---|---|---|---|
| *Volume 200 ml* | | | |
| **1.0:1** | 0.66 | 4.84 | 8.49 |
| **1.2:1** | 1.19 | 6.09 | 14.24 |
| **1.4:1** | 2.65 | 6.55 | 15.21 |
| **1.6:1** | 1.35 | 4.96 | 12.26 |
| **1.8:1** | 2.80 | 6.95 | 14.57 |
| **2.0:1** | 2.20 | 6.88 | 14.79 |
| **2.2:1** | 2.65 | 7.14 | 19.14 |
| **2.4:1** | 1.54 | 7.80 | 22.19 |
| **2.6:1** | 3.02 | 8.11 | 24.67 |
| **2.8:1** | 2.46 | 9.07 | 27.53 |
| **3.0:1** | 1.83 | 9.93 | 30.00 |

**Tables 4** below demonstrates that the yield of fructose complexation is in inverse relation to increasing molar ratio of the carbohydrate to boric acid ratio. Notably, the yield of total fructose in complex as well as the specific yield (total fructose in complex relative to free total fructose) decreased with increasing molar ratio of the carbohydrate to boric acid ratio in counter trend to the yield for di-complex formation. Thus, it should be appreciated that various compositions with high specific di-complex yield at relatively high overall complex yields will typically be achieved where the reaction is performed at a molar ratio of the carbohydrate to boric acid of about 1.6:1 to 2.4:1. Selected results of Table 4 are exemplarily depicted in **Figures 3E and 3F****.**

**Table 4 (not according to the invention)**

| **Molar Ratio** | **Total Fructose in Complex (wt%)** | **Free (wt%)** | **Fructose** | **Complex:Free Ratio** |
|---|---|---|---|---|
| *Volume 200 ml* | | | | |
| **1.0:1** | 86.02 | 13.98 | | 6.15 |
| **1.2:1** | 78.48 | 21.52 | | 3.65 |
| **1.4:1** | 75.59 | 24.41 | | 3.10 |
| **1.6:1** | 81.44 | 18.56 | | 4.39 |
| **1.8:1** | 75.68 | 24.32 | | 3.11 |
| **2.0:1** | 76.14 | 23.86 | | 3.19 |
| **2.2:1** | 71.07 | 28.93 | | 2.46 |
| **2.4:1** | 68.47 | 31.53 | | 2.17 |
| **2.6:1** | 64.20 | 35.80 | | 1.79 |
| **2.8:1** | 60.94 | 39.06 | | 1.56 |
| **3.0:1** | 58.24 | 41.76 | | 1.39 |

Such trend was also observed to be true where the production scale was increased from 200 ml to 1000 ml, to 5,000 ml, and even to 2,000 L as is shown in **Table 5** below and selected results of Table 5 are shown in the graphs of **Figures 4A and 4B****.**

**Table 5 (only Molar Ratio 1.8:1 to 2.4:1 are according to the invention)**

| **Molar Ratio (scale)** | **α-FF-B-Complex Wt%** | **β-FF-B-Complex Wt%** | **β-FP-B-Complex Wt%** | **α-FF Wt%** | **β-FF Wt%** | **β-FP Wt%** |
|---|---|---|---|---|---|---|
| **1:01 (1L)** | 50.6 | 18.4 | 17.0 | 0.7 | 4.8 | 8.5 |
| 1.2:1 **(1L)** | 47.1 | 18.3 | 13.0 | 1.2 | 6.1 | 14.2 |
| **1.4:1 (1L)** | 46.4 | 17.7 | 11.5 | 2.6 | 6.5 | 15.2 |
| **1.6:1 (1L)** | 48.4 | 18.8 | 14.2 | 1.3 | 5.0 | 12.3 |
| **1.8:1 (1L)** | 45.0 | 17.8 | 12.9 | 2.8 | 6.9 | 14.6 |
| **2:01 (1L)** | 44.2 | 18.9 | 13.0 | 2.2 | 6.9 | 14.8 |
| **2.2:1 (1L)** | 43.0 | 18.1 | 10.0 | 2.6 | 7.1 | 19.1 |
| **2.4:1 (1L)** | 41.6 | 17.7 | 9.1 | 1.5 | 7.8 | 22.2 |
| **2.6:1 (1L)** | 39.0 | 17.9 | 7.3 | 3.0 | 8.1 | 24.7 |
| **2.8:1 (1L)** | 37.9 | 16.2 | 6.8 | 2.5 | 9.1 | 27.5 |
| **3:01 (1L)** | 36.0 | 16.5 | 5.7 | 1.8 | 9.9 | 30.0 |
| **1.4:1 (5L)** | 48.8 | 17.5 | 16.3 | 0.9 | 5.9 | 10.6 |
| **1.6:1 (5L)** | 46.5 | 19.2 | 16.3 | 0.9 | 5.9 | 10.6 |
| **1.8:1 (5L)** | 47.8 | 17.6 | 12.9 | 1.5 | 6.2 | 13.9 |
| **2:1 (5L)** | 44.6 | 18.3 | 9.8 | 1.4 | 7.2 | 18.6 |
| **2.2:1 (5L)** | 43.9 | 16.5 | 9.1 | 1.4 | 8.0 | 21.1 |
| **2.0:1 (2000L)** | | | | | | |

**Table 6** further provides experimental data for the yield of total fructose in complex versus free fructose over a variety of molar ratios and production scales.

**Table 6 (only Molar Ratio 1.8:1 to 2.4:1 are according to the invention)**

| **Molar Ratio (scale)** | **Total Fructose in Complex Wt%** | **Free Fructose Wt%** |
|---|---|---|
| **1:01 (1L)** | 86.0 | 14.0 |
| **1.2:1 (1L)** | 78.5 | 21.5 |
| **1.4:1 (1L)** | 75.6 | 24.4 |
| **1.6:1 (1L)** | 81.4 | 18.6 |
| **1.8:1 (1L)** | 75.7 | 24.3 |
| **2:01 (1L)** | 76.1 | 23.9 |
| **2.2:1 (1L)** | 71.1 | 28.9 |
| **2.4:1 (1L)** | 68.5 | 31.5 |
| **2.6:1 (1L)** | 64.2 | 35.8 |
| **2.8:1 (1L)** | 60.9 | 39.1 |
| **3:01 (1L)** | 58.2 | 41.8 |
| **1.4:1 (5L)** | 82.5 | 17.5 |
| **1.6:1 (5L)** | 82.5 | 17.5 |
| **1.8:1 (5L)** | 78.4 | 21.6 |
| **2:1 (5L)** | 72.7 | 27.3 |
| **2.2:1 (5L)** | 69.5 | 30.5 |
| **2.0:1 (2000L)** | 77.8 | 22.2 |

In yet another unexpected result during scale-up of the production volume, the inventors discovered that the production scale had substantial effect on both, the quantities of unreacted boric acid and the yield of di-ester formation for a given molar ratio. In short, and as can be taken from the data in **Table 7**, an increase in production scale at a given molar ratio of fructose to boric acid increased the yield of di-complex, while the same increase in production scale at a given molar ratio of fructose to boric acid decreased unreacted boric acid. Viewed from another perspective, and all other parameters being the same, the inventors discovered that an increase in production scale increased di-complex yield and decreased unreacted boric acid, while leaving the mono-ester substantially unaffected. **Figure 5A** exemplarily shows this trend for unreacted boric acid, while **Figure 5B** illustrates the trend for di-ester formation, and **Figure 5C** shows results for the mono-ester yields. Thus, it should be appreciated that scale-up (with all other parameters being identical) had a rather unexpected technical effect of increasing the yield of di-complex, and decreasing quantities of unreacted boric acid.

**Table 7 (only Molar Ratio 1.8:1 to 2.4:1 and 1L, 5L and 2000L are according to the invention)**

| **Molar Ratio** | **Borate** | | | | **Di-Ester** | | | | **Mono-ester** | | | |
|---|---|---|---|---|---|---|---|---|---|---|---|---|
| | 200 ml | 1 L | 5L | 2000 L | 200 ml | 1 L | 5L | 2000 L | 200 ml | 1 L | 5L | 2000 L |
| **1.0:1** | 44.34 | | | | 47.84 | | | | 7.82 | | | |
| **1.2:1** | 36.42 | | | | 58.26 | | | | 5.32 | | | |
| **1.4:1** | 20.32 | 20.10 | 17.40 | | 73.28 | 73.90 | 74.00 | | 6.40 | 6.00 | 8.60 | |
| **1.6:1** | 13.38 | 12.10 | 11.00 | | 79.12 | 80.20 | 79.80 | | 7.50 | 7.70 | 9.20 | |
| **1.8:1** | 8.46 | 7.90 | 6.80 | | 83.84 | 84.00 | 85.90 | | 7.70 | 8.10 | 7.30 | |
| **2.0:1** | 9.28 | 6.40 | 4.50 | 3.20 | 84.04 | 85.50 | 87.60 | 88.80 | 6.68 | 8.10 | 7.90 | 8.00 |
| **2.2:1** | 6.46 | 4.30 | 5.00 | | 85.86 | 87.50 | 88.50 | | 7.67 | 8.20 | 6.50 | |
| **2.4:1** | 6.64 | | | | 87.01 | | | | 6.35 | | | |
| **2.6:1** | 8.50 | | | | 86.19 | | | | 5.31 | | | |
| **2.8:1** | 10.78 | | | | | 84.33 | | | | | | 4.89 |
| **3.0:1** | 10.31 | | | | | 85.02 | | | | | | 4.68 |

Remarkably, as can be readily appreciated from **Figure 5D**, the scale-dependent increase of the di-ester to boric acid ratio was particularly pronounced for a specific range of molar ratios, while being less pronounced for other molar ratios. More specifically, scale-dependent increase of the di-ester to boric acid ratio was especially evident for the range of molar ratios between 1.8:1 and 2.2:1 as is also reflected in **Table 8** below.

**Table 8 (only 1L, 5L and 2000L are according to the invention)**

| **Molar Ratio** | **Di-ester to Borate Ratio** | | | |
|---|---|---|---|---|
| | 200ml | 1L | 5L | 2000L |
| **1.4** | 3.61 | 3.68 | 4.25 | |
| **1.6** | 5.91 | 6.63 | 7.25 | |
| **1.8** | 9.91 | 10.63 | 12.63 | |
| **2.0** | 9.05 | 13.36 | 19.47 | 27.75 |
| **2.2** | 13.28 | 20.35 | 17.70 | |

Notably, the di-ester to mono-ester ratio was substantially unaffected by the increase in production scale as is reflected in **Figure 5E** and the results of **Table 9** below.

**Table 9 (only 1000ml and 5000ml are according to the invention)**

| **Molar Ratio** | **Di-ester to Mono-ester Ratio** | | |
|---|---|---|---|
| | 200 | 1000 | 5000 |
| **1.4** | 11.45 | 12.32 | 8.60 |
| **1.6** | 10.55 | 10.42 | 8.67 |
| **1.8** | 10.89 | 10.37 | 11.77 |
| **2.0** | 12.58 | 10.55 | 11.09 |
| **2.2** | 11.19 | 10.67 | 13.61 |

Exemplary pH values for various production scales shows the acidity to be substantially uniform in the (mildly) acidic range, generally below 7, in many cases below 6.5, and in most cases at or below 6 as can be seen from **Table 10** below.

**Table 10**

| **Molar Ratio** | **1L** | **5L** | **2000 L** |
|---|---|---|---|
| | pH | pH | pH |
| **1.4:1** | 5.72 | 5.23 | |
| **1.6:1** | 5.73 | 5.64 | |
| **1.8:1** | 5.69 | 5.64 | |
| **2.0:1** | 5.64 | 5.57 | 5.96 |
| **2.2:1** | 5.64 | 5.51 | |

The inventors further investigated whether or not at least water removal from the finished reaction would further affect the product composition. Surprisingly, the inventors discovered that drying (e.g., via freeze-drying "FD" and spray-drying "SD") further dramatically increased di-complex to boric acid ratios in a substantially independent manner of the kind of water removal. Selected exemplary data are provided in **Table 11** below using a single molar ratio of 2:1 (fructose to boric acid) at the listed production scales. Reconstitution was performed with D₂O to original volume prior to water removal (*i.e.,* at a dry ratio of between about 2.2 to 3.5 by weight).

**Table 11 (only 1 liter and 5 liters are according to the invention)**

| **2:1 Molar Ratio Only** | | | | | | | | | |
|---|---|---|---|---|---|---|---|---|---|
| | **200 ml** | | | **1 liter** | | | **5 liters** | | |
| | Liquid | FD | SD | Liquid | FD | SD | Liquid | FD | SD |
| **dry ratio** | N/A | 2.42 | 2.7 | N/A | 3.2 | 3.5 | N/A | 2.6 | 3.3 |
| **Borate** | 9.28 | 5 | 4.7 | 6.4 | 4.3 | 4.1 | 4.5 | 3.7 | 3.9 |
| **Di-complex** | 84.04 | 86.9 | 86.9 | 85.5 | 86.2 | 87.9 | 87.6 | 86.5 | 87.1 |
| **mono-complex** | 6.68 | 8.1 | 8.4 | 8.1 | 8.5 | 8.6 | 7.9 | 9.9 | 9.3 |

**Figure 6A** illustrates the change in di-complex to boric acid ratio as a function of drying method for each of the production volumes, while **Table 12** below and **Figure 6B** show the change in di-complex to boric acid ratio as a function of production volumes for each of the drying methods.

**Table 12 (only 1 liter and 5 liters are according to the invention)**

| **Ratio Di-complex to Borate** | | | | | | | | |
|---|---|---|---|---|---|---|---|---|
| **200 ml** | | | **1 liter** | | | **5 liters** | | |
| Liquid | FD | SD | Liquid | FD | SD | Liquid | FD | SD |
| 9.06 | 17.38 | 18.49 | 13.36 | 20.05 | 21.44 | 19.47 | 23.38 | 22.33 |

Thus, it should be noted that removal of water, and especially drying of the liquid compositions has the unexpected technical effect of substantially increasing the di-complex to unreacted boric acid ratio, with a substantial increase of di-complex and a concomitant decrease in unreacted boric acid.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The numerical values presented in some embodiments of the invention may contain certain errors necessarily resulting from the standard deviation found in their respective testing measurements. Unless the context dictates the contrary, all ranges set forth herein should be interpreted as being inclusive of their endpoints and open-ended ranges should be interpreted to include only commercially practical values. Similarly, all lists of values should be considered as inclusive of intermediate values unless the context indicates the contrary.

The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and docs not pose a limitation on the scope of the invention otherwise claimed. No language in the specification should be construed as indicating any non-claimed element essential to the practice of the invention.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A method of increasing a ratio of di-complex content to unreacted boric acid in a preparative reaction for producing fructoborate complexes, wherein said complexes are prepared by a method comprising:
selecting a molar ratio between a fructose and boric acid of between 1.8:1 and 2.4:1;
reacting the fructose and boric acid in an aqueous solution at the molar ratio to generate a liquid composition comprising fructoborate di-complexes and borate, wherein the preparative scale for the reaction is at least 1000 ml; and
drying the liquid composition to generate a dry product comprising fructoborate di-complexes and borate.

2. The method of claim 1 wherein the 2. preparative scale for the reaction is at least 5,000 mL.

3. The method of claim 1, wherein the molar ratio of sodium fructoborate di-complexes to borate is at least 10:1.

4. The method of any preceding claim, wherein the molar ratio of sodium fructoborate di-complexes to borate is at least 15:1.

5. The method of claim 4, wherein the molar ratio of sodium fructoborate di-complexes to borate is at least 20:1.

6. The method of any preceding claim, wherein the step of drying is performed by freeze-drying.

7. The method of any one of claims 1 to 5, wherein the step of drying is performed by spray drying.

8. The method of any preceding claim, wherein the step of drying decreases unreacted boric acid content of the dry product relative to unreacted boric acid content of the liquid composition.

9. The method of any preceding claim, wherein subsequent to the fructose and boric acid being reacted, and prior to the drying of the liquid composition, the method comprises charge neutralising the fructoborate di-complexes with a cation containing compound, such that the fructoborate di-complexes form a salt with the cation.

10. The method of claim 9, wherein the cation is an alkaline metal hydroxide, an alkaline earth metal hydroxide, an alkaline metal carbonate or an alkaline earth metal carbonate.

11. The method of claim 9 or 10, wherein the aqueous solution is at an acidic pH.

12. The method of claim 11, wherein the acidic pH is a pH of less than 6.0.

13. The method of any preceding claim, wherein the fructose and boric acid are reacted for at least 30 minutes.

## Patentansprüche

1. Verfahren zum Erhöhen eines Verhältnisses von Dikomplexgehalt zu nicht umgesetzter Borsäure in einer präparativen Reaktion zum Produzieren von Fructoboratkomplexen, wobei die Komplexe durch ein Verfahren hergestellt werden, umfassend:
Auswählen eines Molverhältnisses zwischen einer Fructose und Borsäure zwischen 1,8:1 und 2,4:1;
Umsetzen der Fructose und Borsäure in einer wässrigen Lösung in dem Molverhältnis, um eine flüssige Zusammensetzung zu erzeugen, die Fructoborat-Dikomplexe und Borat umfasst, wobei der präparative Maßstab für die Umsetzung zumindest 1000 ml ist; und
Trocknen der flüssigen Zusammensetzung, um ein Trockenprodukt zu erzeugen, das Fructoborat-Dikomplexe und Borat umfasst.

2. Verfahren nach Anspruch 1, wobei der präparative Maßstab für die Umsetzung zumindest 5.000 ml ist.

3. Verfahren nach Anspruch 1, wobei das Molverhältnis von Natriumfructoborat-Dikomplexen zu Borat zumindest 10:1 ist.

4. Verfahren nach einem vorhergehenden Anspruch, wobei das Molverhältnis von Natriumfructoborat-Dikomplexen zu Borat zumindest 15:1 ist.

5. Verfahren nach Anspruch 4, wobei das Molverhältnis von Natriumfructoborat-Dikomplexen zu Borat zumindest 20:1 ist.

6. Verfahren nach einem vorhergehenden Anspruch, wobei der Schritt des Trocknens durch Gefriertrocknen durchgeführt wird.

7. Verfahren nach einem der Ansprüche 1 bis 5, wobei der Schritt des Trocknens durch Sprühtrocknen durchgeführt wird.

8. Verfahren nach einem vorhergehenden Anspruch, wobei der Schritt des Trocknens Gehalt an nicht umgesetzter Borsäure des Trockenprodukts relativ zu Gehalt an nicht umgesetzter Borsäure der flüssigen Zusammensetzung verringert.

9. Verfahren nach einem vorhergehenden Anspruch, wobei anschließend daran, dass die Fructose und Borsäure umgesetzt werden, und vor dem Trocknen der flüssigen Zusammensetzung das Verfahren Ladungsneutralisieren der Fructoborat-Dikomplexe mit einer Kationen enthaltenden Verbindung umfasst, sodass die Fructoborat-Dikomplexe ein Salz mit dem Kation bilden.

10. Verfahren nach Anspruch 9, wobei das Kation ein Alkalimetallhydroxid, ein Erdalkalimetallhydroxid, ein Alkalimetallcarbonat oder ein Erdalkalimetallcarbonat ist.

11. Verfahren nach Anspruch 9 oder 10, wobei die wässrige Lösung bei einem sauren pH ist.

12. Verfahren nach Anspruch 11, wobei der saure pH ein pH von weniger als 6,0 ist.

13. Verfahren nach einem vorhergehenden Anspruch, wobei Fructose und Borsäure zumindest 30 Minuten umgesetzt werden.

## Revendications

1. Procédé permettant d'augmenter le rapport de la teneur en di-complexes à l'acide borique n'ayant pas réagi dans une réaction préparative pour produire des complexes de fructoborate, lesdits complexes étant préparés par un procédé comprenant :
la sélection d'un rapport molaire entre un fructose et l'acide borique compris entre 1,8:1 et 2,4:1 ;
la réaction du fructose et de l'acide borique dans une solution aqueuse au rapport molaire pour générer une composition liquide comprenant des di-complexes de fructoborate et du borate, l'échelle préparative pour la réaction étant d'au moins 1000 ml ; et
le séchage de la composition liquide pour générer un produit sec comprenant des di-complexes de fructoborate et du borate.

2. Procédé selon la revendication 1, ladite échelle préparative pour la réaction étant d'au moins 5 000 ml.

3. Procédé selon la revendication 1, ledit rapport molaire des di-complexes de fructoborate de sodium au borate étant d'au moins 10:1.

4. Procédé selon l'une quelconque des revendications précédentes, ledit rapport molaire des di-complexes de fructoborate de sodium au borate étant d'au moins 15:1.

5. Procédé selon la revendication 4, ledit rapport molaire des di-complexes de fructoborate de sodium au borate étant d'au moins 20:1.

6. Procédé selon l'une quelconque des revendications précédentes, ladite étape de séchage étant réalisée par lyophilisation.

7. Procédé selon l'une quelconque des revendications 1 à 5, ladite étape de séchage étant réalisée par séchage par pulvérisation.

8. Procédé selon l'une quelconque des revendications précédentes, ladite étape de séchage diminuant la teneur en acide borique n'ayant pas réagi du produit sec par rapport à la teneur en acide borique n'ayant pas réagi de la composition liquide.

9. Procédé selon l'une quelconque des revendications précédentes, après la réaction du fructose et de l'acide borique, et avant le séchage de la composition liquide, ledit procédé comprenant la neutralisation de charge des di-complexes de fructoborate avec un composé contenant un cation, de sorte que les di-complexes de fructoborate forment un sel avec le cation.

10. Procédé selon la revendication 9, ledit cation étant un hydroxyde de métal alcalin, un hydroxyde de métal alcalino-terreux, un carbonate de métal alcalin ou un carbonate de métal alcalino-terreux.

11. Procédé selon la revendication 9 ou 10, ladite solution aqueuse étant à un pH acide.

12. Procédé selon la revendication 11, ledit pH acide étant un pH inférieur à 6,0.

13. Procédé selon l'une quelconque des revendications précédentes, ledit fructose et ledit acide borique réagissant pendant au moins 30 minutes.
